(19) Europäisches Patentamt
European Patent Office
Office européen des brevets

(11) Publication number: **0 332 241 B1**

(12) EUROPEAN PATENT SPECIFICATION

(45) Date of publication of patent specification: **04.01.95**

(51) Int. Cl.⁶: **A61K 39/295**, A61K 39/215, //A61K39/17,A61K39/12, A61K39/235,A61K39/255, A61K39/125,A61K39/245, A61K39/21,A61K39/15

(21) Application number: **89200382.3**

(22) Date of filing: **17.02.89**

The file contains technical information submitted after the application was filed and not included in this specification

(54) **Live combination vaccine.**

(30) Priority: **08.03.88 US 165339**

(43) Date of publication of application:
**13.09.89 Bulletin 89/37**

(45) Publication of the grant of the patent:
**04.01.95 Bulletin 95/01**

(84) Designated Contracting States:
**AT BE CH DE ES FR GB GR IT LI NL SE**

(56) References cited:
**EP-A- 0 086 025**
**EP-A- 0 212 703**
**US-A- 2 798 835**

**J. Gen. Virol., vol. 28 (1975); R.W. BINGHAM et al., pp. 381-390;**

(73) Proprietor: **Akzo Nobel N.V.**
**Velperweg 76**
**NL-6824 BM Arnhem (NL)**

(72) Inventor: **Schrier, Carla Christina**
**Mees 29**
**NL-5831 MR Boxmeer (NL)**

(74) Representative: **Hermans, Franciscus G.M. et al**
**Patent Department**
**AKZO NOBEL N.V.**
**Pharma Division**
**P.O. Box 20**
**NL-5340 BH Oss (NL)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

The invention is concerned with live combination vaccines containing infectious bronchitis viruses.

Infectious Bronchitis Virus (IBV) is a Corona virus which is infectious to birds and in particular to poultry. Infection with the virus results in an acute respiratory disease, characterized by tracheal rales, coughing, sneezing and nasal discharge. Infectious bronchitis (i.b.) may cause a considerable mortality among afflicted chickens, and moreover may damage their kidneys. In layer and in breeder hens the infection may cause a drop in egg production due to damage of the reproductive tract. In many cases the egg drop is accompanied by an enteritis causing diarrhoea.

Poultry can be protected against IBV infections by vaccination. Moreover, there is a desire to protect the birds at as early an age as possible. These young chickens are also vulnerable to infection by other viruses, such as Newcastle Disease Virus (NDV), Infectious Bursal Disease Virus (IBDV), Marek's Disease Virus (MDV), other avian Herpes Viruses, Fowl Pox Virus (FPV), Avian Encephalomyelitis Virus (AEV), Reticulo-endoteleosis Virus (REV), avian Adeno Viruses and avian Reo Viruses. Of course, there is a desire to protect the young chickens not only against IBV infection but to protect them against infections by other of the above-mentioned viruses as well. To this end, young chickens have been vaccinated with vaccines comprising combinations of immunogens derived from IBV as well as from at least one of the other viruses. These vaccines may contain live or inactivated viruses, but in principle live vaccines are favored. A well known problem with these live combination vaccines is the mutual influence of the antigenic components resulting in a decrease of the potency of one or more of the constituting components. This problem has been reported for the combination IBV/NDV by e.g. Raggi & Lee (1964) Avian Disease 8, 471-480; Hanson & Alberts (Am.J.Vet.Res. March 1959, 352-356) and Thornton & Muskett (The Veterinary Record, May 1975, 467-468); conflicting results were published by Winterfield (Poultry Science 1984, 63, 182-184).

The live vaccine of the present invention is characterized in that it is derived from Infectious Bronchitis virus strain Ma5, deposited with the ATCC, at Rockville, Md., U.S.A. under accession No. VR 2199.

It has also been found now that the shortcomings of the known live combined virus vaccines described above can be overcome by employing the novel vaccine according to the present invention.

IBV's having the property to spontanously hemagglutinate chicken erythrocytes are known to possess superior vaccinating properties from the European patent publication No. 0.086.025. Also, the use of these particular IBV's in inactivated combined vaccines was disclosed in this latter publication.

European patent application 0.212.703 published 04.03.87 discloses a live IBV vaccine which is safe for use in day-old chickens derived from an IBV strain of the D207 serotype.

US patent no. 2.798.835 published 09.07.57 discloses a live IBV vaccine derived from IBV strain D-G.

According to the invention the vaccine may comprise at least one other virus infectious to poultry but different from IBV.

This latter so-called other virus can be selected from, for example, NDV, IBDV, MDV, other avian Herpes Viruses, FPV, avian Adeno Viruses and avian Reo Viruses.

The IBV strain for use according to the present invention is strain Ma5 (working seed of which is deposited on December 15, 1987 under no. VR 2199 with ATCC at Rockville, USA). Strains Ma5 which belongs to the Massachusetts serotype.

The viruses for use in the preparation of the vaccines according to the present invention can be grown in any medium suitable for culture of avian viruses. Particularly suitable is the growing of these viruses in embryonated SPF chicken eggs or on a cell culture, preferably from avian tissues.

Thus grown viruses can be brought together in the desired ratios and the resulting mixture can be divided into quantities suitable for vaccination of either a single bird or a multitude of birds.

The live vaccine may be prepared in the form of a suspension or may be lyophilized.

In lyophilized vaccines it is preferable to add one or more stabilizers. Suitable stabilizers are for example SPGA (described by Bovarnick (1950) J.Bacteriology 59; 509), carbohydrates (such as sorbitol, mannitol, starch, sucrose, dextran, glucose), proteins (such as albumin or casein) or degradation products thereof, protein containing agents (such as bovine serum or skimmed milk) and buffers (such as alkali metal phosphates). Optionally, one or more compounds having adjuvant activity may be added too. Suitable adjuvants are for example aluminium hydroxide, phosphate or oxide, mineral oils (such as Bayol F®, Marcol 52® and saponins.

Optionally the viruses can be attenuated. This attenuation can be performed by adaptation of the viruses to embryonated eggs or a cell culture (preferably chicken embryo kidney cells) and passaging the virus in those cultures e.g. 10 to 200 times.

The live vaccines according to the invention may be administered for example by muscular, subcutaneous or in ovo injection, eye drop, nose drop, drinking water or spray methods, and preferably at an age varying from one day old to the point of lay (about 18 weeks).

For administration to one-day-old chickens advantageously use may be made of vaccines containing the mentioned Ma5 strain or a strain derived therefrom which also shows spontaneous hemagglutination of chicken erythrocytes .

For live vaccines a dosage of each of the respective virus strains may be used in a range of $3 \log EID_{50}$ to $7 \log EID_{50}$ per bird, preferably between about $4 \log EID_{50}$ to $5 \log EID_{50}$ per bird.

Example I

**Comparison of two live NDV/IBV combination vaccines and separate vaccines of the two respective viruses.**

Experimental design: one-day-old SPF chickens were divided into 8 groups and placed in negative pressure isolators.

Group I was vaccinated by eye-drop with $10^4$ $EID_{50}$/bird of the Ma5 strain ($HA^+$ strain).

Group II was vaccinated by eye-drop with $10^4$ $EID_{50}$/bird of the IBV-Mildvac-M (a commercial available vaccine for one-day-old broiler chickens produced by Intervet America Inc.; $HA^-$ strain).

Group III was vaccinated by eye-drop with minimal $10^{5.8}$ $EID_{50}$/bird of the NDV-B1 strain (a commercial available vaccine of a Hitchner type NDV strain - Intervet America Inc.).

Groups IV and V were vaccinated by eye-drop with a combination of the IBV-Mildvac-M and the NDV-B1 strain (with the same dose as in the single vaccination).

Groups VI and VII were vaccinated by eye-drop with a combination of the Ma5 strain and the NDV-B1 strain (with the same dose as in the single vaccination).

Group VIII was vaccinated by eye-drop with negative allantoic-fluid and served as controls.

Challenge was performed at 21 post-vaccination with either the USDA-IBV-M41-challenge strain (group I, II, IV, VI and part of VIII) or the USDA-NDV-challenge strain (group III, V, VII and part of VIII).

From the birds which received a challenge with the USDA-IBV-M41-challenge strain, clinical symptoms were recorded four days post-challenge; the birds were then killed and tracheas were collected for virus recovery attempts and for observation of cilial movement. The birds which received a challenge with the USDA-NDV-challenge strain were observed for mortality and clinical signs during 10 days post-challenge.

**Results:**

In table 1 percentile protection against USDA-IBV-M41-challenge strain as measured by virus recovery, ciliostasis and clinical signs four days post-challenge are given.

Table 1

| Vaccine | Ciliostasis four days p.c. | Virus recovery four days p.c. | Clinical signs four days p.v. |
|---|---|---|---|
| Ma5 | 95* | 60* | 100* |
| Mildvac-M | 85 | 60 | 100 |
| Ma5/NDV-B1 | 85 | 70 | 100 |
| Mildvac-M/NDV-B1 | 70 | 15 | 80 |
| Negative allantoic fluid | 0 | 0 | 70 |

\* = percentage of birds protected against challenge three weeks post- vaccination as measured by different criteria.

The Ma5 ($HA^+$) strain, in combination with a NDV-strain gives a better protection against a challenge with a M41-challenge strain than the Mildvac-M ($HA^-$) strain. No significant difference in protection could be observed between the groups which received a NDV challenge. The protection in all cases was sufficient: more than 90% of the chicks were protected in all groups.

Example II

One day old commercial broiler chickens were divided in five groups and placed in isolators.

2 groups were vaccinated with the combination H120/Clone 30 (one group by coarse spray, the other group by eye-drop) and 2 other groups were vaccinated with the combination Ma5/Clone 30 (one group by coarse spray; one group by eye-drop); one group remained as unvaccinated controls.

Clone 30 is a NDV vaccine, commercially available from Intervet .

Four and eight days post-vaccination 6 birds per group were removed, clinical symptoms were recorded and their tracheas were examined in the ciliostasis test.

Five weeks post-vaccination the birds vaccinated by eye-drop were submitted to a challenge with the M41-challenge strain ($10^6$ $EID_{50}$ per bird, eye-drop). Four days after the M41-challenge, clinical symptoms were recorded and tracheas were taken for the ciliostasis test.

The birds vaccinated by course spray were submitted to a NDV-challenge five weeks post-vaccination (NDV-Herts-challenge strain, $10^7$ $EID_{50}$ per bird; intramuscular) and clinical symptoms and mortality were recorded daily during 12 days.

Results: Tables 2, 3, 4 and 5 show the results of the above experiments. It is clear, that the combination with the spontaneously hemagglutinating IB strain (Ma5) provides better protection against challenge with both a M41-challenge strain and a NDV-challenge strain than the combination with the IB strain which is not spontaneously hemaglutinating chicken erythrocytes (H120).

## Table 2

## CLINICAL SYMPTOMS POST VACCINATION

- VACCINATION AT ONE DAY OLD

- CLINICAL SYMPTOMS (SNEEZING, GURGLING) AT FOUR AND EIGHT DAYS POST-VACCINATION (P.V.)

TITRE AT DAY OF VACCINATION EXPRESSED AS HI $^2$LOG; M41 7.6; NDV 5.2.

| | PERCENTAGE OF BIRDS WITH CLINICAL SYMPTOMS | |
|---|---|---|
| VACCINE | FOUR DAYS P.V. | EIGHT DAYS P.V. |
| H120 (HA$^-$)/CLONE 30 EYE-DROP | 0 | 33 |
| H120 (HA$^-$)/CLONE 30 SPRAY | 0 | 17 |
| MA5 (HA$^+$)/CLONE 30 EYE-DROP | 0 | 17 |
| MA5 (HA$^+$)/CLONE 30 SPRAY | 0 | 0 |
| NONE | 0 | 0 |

## Table 3

### CILIA STOPPING TEST POST VACCINATION

- VACCINATION AT ONE DAY OLD
- CILIA STOPPING TEST AT FOUR AND EIGHT DAYS POST-
  VACCINATION (P.V.)

TITRE AT DAY OF VACCINATION AS HI $^2$LOG; M41 7.6; NDV 5.2

| | PERCENTAGE OF BIRDS WITH CILIA STOPPING | |
|---|---|---|
| VACCINE | FOUR DAYS P.V. | EIGHT DAYS P.V. |
| H120 (HA$^-$)/CLONE 30 EYE-DROP | 17 | 33 |
| H120 (HA$^-$)/CLONE 30 SPRAY | 0 | 17 |
| MA5 (HA$^+$)/CLONE 30 EYE-DROP | 0 | 0 |
| MA5 (HA$^+$)/CLONE 30 SPRAY | 0 | 17 |
| NONE | 0 | 0 |

## Table 4

### PROTECTION COMBINED VACCINE AGAINST ND

- VACCINATION AT ONE DAY OLD
- CHALLENGE WITH A NDV-CHALLENGE STRAIN FIVE WEEKS P.V.

|  | CHALLENGE WITH NDV-CHALLENGE STRAIN |
|---|---|
| VACCINE | PERCENTAGE OF ANIMALS PROTECTED |
| H120 (HA$^-$)/CLONE 30 SPRAY | 83 |
| MA5 (HA$^+$)/CLONE 30 SPRAY | 100 |
| NONE | 0 |

## Table 5

### PROTECTION COMBINED VACCINE AGAINST IB

- VACCINATION AT ONE DAY OLD
- CHALLENGE WITH A M41 CHALLENGE STRAIN FIVE WEEKS P.V.

|  | FOUR DAYS POST CHALLENGE WITH A M41-CHALLENGE STRAIN | |
|---|---|---|
| VACCINE | PERCENTAGE OF ANIMALS WITH CLINICAL SYMPTOMS POST-CHALLENGE | PERCENTAGE OF ANIMALS PROTECTED AS MEASURED IN THE CILIOSTASIS TEST |
| H120 (HA$^-$)/CLONE 30 EYE-DROP | 30 | 58 |
| MA5 (HA$^+$)/CLONE 30 EYE-DROP | 0 | 100 |
| NONE | 83 | 0 |

Example III

Two hundred and fifty one-day-old SPF chickens (SPAFAS®) were divided into four groups each containing fifty chickens and placed into negative pressure isolators. The birds were then vaccinated with the following strains:

group I : Ma5;n $10^4$ $EID_{50}$/animal (eye-drop) HA$^+$ strain

group II : Mildvac-M (Mass-type; B48 strain, commercially available from Intervet America Inc.); $10^4$ $EID_{50}$/animal (eye-drop); HA$^-$ strain

EP 0 332 241 B1

group III : IB-vac-M (Mass-type; Connaught strain, commercially available from Intervet America Inc.); $10^4$ EID$_{50}$/animal (eye-drop); HA$^-$ strain
group IV : Negative allantoic fluid; 0.1 ml/animal (eye-drop).

Five weeks post vaccination all groups were submitted to a challenge with the M41-challenge strain. Five days post challenge clinical symptoms from ten birds per group were recorded, the birds were then killed and tracheas were removed for virus reisolation and observation of ciliary activity. Seven days post-challenge the remaining birds in all groups were necropsied.

**Results**

Table 6

| Recovery of challenge virus 5 days p.c. | | |
|---|---|---|
| Vaccine | Virus recovery | % protected |
| Ma5 | 0/10* | 100 |
| Mildvac | 2/10 | 80 |
| IB-vac-M | 0/10 | 100 |
| Negative AAF | 10/10 | 0 |

* ratios express number of birds yielding virus per total number of birds.

Table 7

| Clinical symptoms 5 days p.c. | |
|---|---|
| Vaccine | % of birds with clinical symptoms p.c. |
| Ma5 | 0 |
| Mildvac-M | 20 |
| IB-vac-M | 10 |
| Negative AAF | 90 |

Table 8

| Ciliostasis test 5 days p.c. | | |
|---|---|---|
| Vaccine | +/total | % protected |
| Ma5 | 5/5* | 100 |
| Mildvac-M | 4/5 | 80 |
| IB-vac-M | 5/5 | 100 |
| Negative AAF | 0/5 | 0 |

* ratios express number of animals wih a mean ciliary activity greater than 2 per total number of birds.

7

Table 9

| Necropsy results 7 days p.c. | |
|---|---|
| Vaccine | % of birds with air sacculitis |
| Ma5 | 0 |
| Mildvac-M | 0 |
| IB-vac-M | 16.6 |
| Negative AAF | 42.8 |

Conclusion: The Ma5 strain, which displays spontaneous hemagglutination gives a better protection against challenge than the two HA$^-$ strains compared belonging to the same serotype. Furthermore, in experiments not shown in the present example it was found that the Ma5 strain has outstanding properties also in combination vaccines together with other IBV strains.

Example IV

This experiment was conducted to compare the immunogenicity of a spontaneously haemagglutinating IBV-Massachusetts strain (Ma5) and a non-haemagglutinating IBV-Massachusetts strain (H120) when administered in combination with an ILT (Infectious Laryngotracheitis)-vaccine.

Materials and methods:

Vaccines

The following vaccine strains were used for the experiment:
1. IBV-Ma5
The vaccine was diluted 1:100 in tryptose phosphate broth and 0.1 ml was administered to each bird by eye-drop (titre : $10^{4.6}$ $EID_{50}$ of the Ma5-strain per bird).
2. IBV-H120
The Vaccine was diluted 1:100 in tryptose phosphate broth and 0.1 ml of the diluted vaccine was administered to each bird by eye-drop (titre : $10^{5.1}$ $EID_{50}$ of the H120-strain per bird).
3. ILT-vaccine.
The vaccine was diluted according to the instruction of the manufacturer and each bird received one bird dose by eye-drop.

Experimental design:

Four weeks old S.P.F.-birds were divided in three groups and placed in negative pressure isolators.
One group was vaccinated by eye-drop with one bird dose of the Ma5-vaccine and one bird dose of the ILT-vaccine. The third group remained as controls. Four weeks post-vaccination the birds were subjected to a challenge with an IBV-M41 challenge strain (M41-8th passage (Weybridge strain); $10^{4.8}$ $EID_{50}$ per bird; eye-drop).
Four days post-challenge the birds were killed and their tracheas were taken for examination of the ciliary activity and for virus recovery attempts.

Results:

As shown in table 1 100% of the birds in the Ma5-ILT vaccinated group and 64% of the birds in the H120-ILT vaccinated group were protected against a challenge with a M41-challenge strain as measured in the ciliostatis test.
Challenge virus could be recovered from 36% of the birds vaccinated with the Ma5-ILT combination and from all birds vaccinated with the H120-ILT combination.

Conclusion:

The protection obtained with the spontaneously haemagglutinating IBV-strain Ma5 in combination with an ILT-vaccine is superior to the protection obtained with a non-haemagglutinating IBV-strain (like the H120) in combination with a ILT-vaccine.

Table 10

| Efficacy of a combined Ma5-ILT-vaccine and a combined H120-ILT-vaccine in SPF-chickens vaccinated at four weeks of age and challenged at eight weeks of age with a M41-challenge strain. | | |
|---|---|---|
| Vaccine | % protection as measured by | |
| | ciliostatis test | virus recovery |
| Ma5/ILT | 100 (N-11) | 64 (N-11) |
| H120/ILT | 64 (N-11) | 0 (N-11) |
| Controls | 0 (N-7) | 0 (N-7) |

**Claims**
**Claims for the following Contracting States : AT, BE, CH, DE, FR, GB, IT, LI, NL, SE**

1. A live vaccine for immunizing poultry against Infectious Bronchitis Virus, derived from Infectious Bronchitisvirus strain Ma5, deposited with the ATCC, at Rockville, Md, U.S.A. under accession no. VR 2199.

2. A vaccine according to claim 1, characterised in that it additionally comprises at least one other virus infectious to poultry but different from Infectious Bronchitis Virus.

3. A vaccine according to claim 2 characterised in that the other virus is Newcastle Disease Virus or Infectious Laringotracheitis Virus.

4. An essentially pure culture of Infectious Bronchitis Virus belonging to the Ma5 strain deposited with the ATCC, at Rockville, Md, U.S.A. under accession no. VR 2199 or a spontaneously haemagglutinating strain derived thereof.

5. A process for the preparation of a combined vaccine according to claim 2 comprising bringing together Infectious Bronchitisvirus VR 2199 or a spontaneously haemagglutinating strain derived thereof and at least one other virus infectious to poultry but different from Infectious Bronchitis Virus.

**Claims for the following Contracting States : ES, GR**

1. Method for the preparation of a live vaccine for immunising poultry against Infectious Bronchitis virus, comprising mixing Infectious Bronchitis virus strain Ma5, deposited with the ATCC at Rockville, Md, U.S.A. under accession no VR 2199 or a spontaneously haemagglutinating strain derived thereof, and a physiologically acceptable carrier.

2. Method for the preparation of a live vaccine according to claim 1, characterised in that at least one other virus infectious to poultry but different from Infectious Bronchitis virus is mixed in addition.

3. Method for the preparation of a live vaccine according to claim 2, characterised that the other virus, mixed in addition, is Newcastle Disease virus or Infectious Laringotracheitis virus.

4. Method for the preparation of an essentially pure culture of Infectious Bronchitis virus belonging to the Ma5 strain deposited with the ATCC, deposited with the ATCC at Rockville, Md, U.S.A. under accession no VR 2199 or a spontaneously haemagglutinating strain derived thereof, comprising growing said virus on suitable cells.

**Patentansprüche**

**Patentansprüche für folgende Vertragsstaaten : AT, BE, CH, DE, FR, GB, IT, LI, NL, SE**

1. Lebendimpfstoff zum Immunisieren von Geflügel gegen infektiösen Bronchitis-Virus, abgeleitet vom infektiösen Bronchitis-Virus-Stamm Ma5, hinterlegt bei ATCC in Rockville, Md, USA unter der Zulassungsnummer VR 2199.

2. Impfstoff nach Anspruch 1, dadurch gekennzeichnet, dass er zusätzlich mindestens einen anderen, für Geflügel ansteckenden Virus enthält, der aber vom infektiösen Bronchitis-Virus verschieden ist.

3. Impfstoff nach Anspruch 2, dadurch gekennzeichnet, dass das andere Virus das Newcastle-Disease-Virus oder das infektiöse Laringotracheitis-Virus ist.

4. Eine im wesentlichen reine Kultur von infektiösem Bronchitis-Virus, der zu dem bei ATCC in Rockville, Md, USA unter der Zulassungsnummer VR 2199 hinterlegten Stamm Ma5 oder einem spontan blutgerinnenden, davon abgeleiteten Stamm gehört.

5. Verfahren zur Herstellung eines kombinierten Impfstoffes nach Anspruch 2, das das Zusammenbringen von infektiösem Bronchitis-Virus VR 2199 oder einem davon abgeleitetem, spontan blutgerinnendem Stamm und mindestens einem anderen, für Geflügel infektiösem Virus, der aber vom infektiösem Bronchitis-Virus verschieden ist, umfasst.

**Patentansprüche für folgende Vertragsstaaten : ES, GR**

1. Verfahren zur Herstellung eines Lebendimpfstoffs zur Immunisierung von Geflügel gegen infektiösen Bronchitis-Virus, das das Vermischen von infektiösem Bronchitis-Virus, Stamm Ma5, hinterlegt bei ATCC in Rockville, Md, USA unter der Zulassungsnummer VR 2199 oder einem davon abgeleiteten, spontan blutgerinnenden Stamm mit einem physiologisch annehmbaren Träger umfasst.

2. Verfahren zur Herstellung eines Lebendimpfstoffs nach Anspruch 1, dadurch gekennzeichnet, dass zusätzlich ein anderes, für Geflügel ansteckendes, aber von infektiösem Bronchitis-Virus verschiedenes Virus beigemischt wird.

3. Verfahren zur Herstellung eines Lebendimpfstoffs nach Anspruch 2, dadurch gekennzeichnet, dass das andere, zusätzlich beigemischte Virus das Newcastle-Disease-Virus oder das infektiöse Laringotracheitis-Virus ist.

4. Verfahren zur Herstellung einer im wesentlichen reinen Kultur von infektiösem Bronchitis-Virus, welcher zu dem bei ATCC in Rockville, Md, USA unter der Zulassungsnummer VR 2199 hinterlegten Stamm Ma5 oder einem davon abgeleiteten, spontan blutgerinnenden Stamm gehört, welches das Züchten dieses Virus auf geeigneten Zellen umfasst.

**Revendications**

**Revendications pour les Etats contractants suivants : AT, BE, CH, DE, FR, GB, IT, LI, NL, SE**

1. Un vaccin vivant pour immuniser les volailles contre le virus de la bronchite infectieuse, dérivé de la souche Ma5 du virus de la bronchite infectieuse, déposée auprès de l'ATCC à Rockville, Md, U.S.A., sous le numéro d'accès VR 2199.

2. Un vaccin selon la revendication 1, caractérisé en ce qu'il comprend de plus au moins un autre virus infectieux pour les volailles mais différent du virus de la bronchite infectieuse.

3. Un vaccin selon la revendication 2, caractérisé en ce que l'autre virus est le virus de la maladie de Newcastle ou le virus de la laryngotrachéite infectieuse.

4. Une culture essentiellement pure du virus de la bronchite infectieuse appartenant à la souche Ma5 déposée auprès de l'ATCC à Rockville, Md, U.S.A., sous le numéro d'accès VR 2199 ou d'une souche en dérivant entraînant une hémagglutination spontanée.

**5.** Un procédé de préparation d'un vaccin combiné selon la revendication 2 comprenant la combinaison du virus de la bronchite infectieuse VR 2199, ou d'une souche en dérivant entraînant une hémagglutination spontanée, avec au moins un autre virus infectieux pour les volailles différent du virus de la bronchite infectieuse.

**Revendications pour les Etats contractants suivants : ES, GR**

**1.** Procédé de préparation d'un vaccin vivant pour immuniser les volailles contre le virus de la bronchite infectieuse, comprenant le mélange d'un souche Ma5 du virus de la bronchite infectieuse, déposée auprès de l'ATCC à Rockville, Md, U.S.A., sous le numéro d'accès VR 2199, ou d'une souche en dérivant entraînant une hémagglutination spontanée, avec un support physiologiquement acceptable.

**2.** Procédé de préparation d'un vaccin vivant selon la revendication 1, caractérisé en ce qu'au moins un autre virus infectieux pour les volailles mais différent du virus de la bronchite infectieuse est mélangé en plus.

**3.** Procédé de préparation d'un vaccin vivant selon la revendication 2, caractérisé en ce que l'autre virus mélangé en plus est le virus de la maladie de Newcastle ou le virus de la laryngotrachéite infectieuse.

**4.** Procédé de préparation d'une culture essentiellement pure du virus de la bronchite infectieuse appartenant à la souche Ma5 déposée auprès de l'ATCC à Rockville, Md, U.S.A., sous le numéro d'accès VR 2199, ou à une souche en dérivant entraînant une hémagglutination spontanée, comprenant la culture dudit virus sur des cellules appropriées.